# EUROPEAN PATENT APPLICATION

(11) **EP 1 602 644 A1**
(43) Date of publication of application: **07.12.2005**
(21) Application number: 04717651.6
(22) Date of filing: 05.03.2004
(51) Int. Cl.: C07C 309/35, C07D 215/36, A61K 31/185, A61K 31/47

(54) **PHARMACEUTICAL COMPOSITIONS CONTAINING SULPHONIC ACID DERIVATIVES**

(30) Priority: 07.03.2003 ES 200300563
(71) Applicant: Italfarmaco, S.A., 28108 Alcobendas (Madrid) (ES); CONSEJO SUPERIOR DE INVESTIGACIONES CIENTIFICAS, 28006 Madrid (ES)
(72) Inventor: BANFITOSI, Beatriz, Maria, E-28108 Alcobendas (Madrid) (ES); GIMENEZ GALLEGO, Guillermo, E-28006 Madrid (ES); VALVERDE LOPEZ, Serafin, E-28006 Madrid (ES); LOZANO PUERTO, Rosa Maria, E-28006 Madrid (ES); CUEVAS SANCHEZ, Pedro, E-28034 Madrid (ES)
(74) Representative: de Justo Vàzquez, Jorge M.
(86) International application number: PCT/ES2004/000104
(87) International publication number: WO 2004/078704

(57) **Abstract**

A naphthalenesulfonic acid or quinolinesulfonic acid of formula (I), wherein A is N or a CR⁸ formula group, where R⁸ is H, OH, NR¹⁰R¹¹, independently from one another, where R¹⁰ and R¹¹ represent H or C₁-C₆ alkyl or a group of formula NH-CO-R¹², where R¹² is C₁-C₆ alkyl or C₆-C₁₀ aryl; R¹ and R² represent H or SO₃R⁹, independently from one another, where R⁹ is H, ammonium or a cation of an alkali or alkaline-earth metal; R³ is H or OH; and R⁴, R⁵, R⁶ and R⁷ represent H, an NR¹⁰R¹¹ or NH-CO-R¹² group, independently from one another; on the condition that (i) at least one of R¹ or R² is SO₃R⁹, and (ii) at least one of R⁴, R⁵, R⁶ or R⁷ is an NR¹⁰R¹¹ or NH-CO-R¹² group, or their pharmaceutically acceptable salts; and a pharmaceutically acceptable excipient.

## Description

### FIELD OF THE INVENTION

This invention refers to pharmaceutical compositions comprising at least one naphthalenesulfonic or quinolinesulfonic acid derivative and to their therapeutic and/or diagnostic applications.

### BACKGROUND OF THE INVENTION

Acid and base fibroblast growth factors (aFGF and bFGF, respectively) are two polypeptides the biochemical and biological properties of which are very similar and are considered paradigmatic for the entire mitogen family (FGFs) they belong to. FGFs typically show a strong affinity for heparin and for the glycoside portion of heparan sulfate, it having been shown that the binding to any of these polysulfates is required for the FGFs to recognize their specific tyrosine kinase receptor on the cell surface and to transduce their presence in a cell division signal.

FGFs are very important angiogenesis promoters and inappropriate FGF expression could contribute to develop cancers and other types of pathologies. Angiogenesis is a process characterized by the formation of new blood vessels in a tissue or organ taking place in certain normal physiological situations, for example, in wound healing, in fetal and embryonic development and in the formation of the *corpus luteum,* the endometrium and the placenta. Angiogenesis further constitutes the etiological basis of certain pathological conditions, for example, cancer, diabetic retinopathy, rheumatoid arthritis and the like. Therefore, it is believed that antiangiogenesis, particularly FGF inhibition activity, may be a form of pharmacological treatment for these diseases, particularly for cancer, and especially for solid tumors. When solid tumors become malignant they induce the formation of dense vascular networks by means of which they receive the supplies required for growth and they eliminate their catabolism products. The collapse of the tumor is caused by preventing the formation of this vascular network due to the lack of nutrients and autointoxication.

In addition to the antiangiogenic effect, FGF activity inhibition has other potentially positive effects in antitumor therapy. Regardless of their angiogenic activity, FGFs directly induce the proliferation of numerous types of tumor cells [e.g. Bieker R. et al. (2003) *Cancer Res.* 63, 7241-7246; Polnaszek N. et al. (2003) *Cancer Res.* 63, 5754-5760; Kono K. et al, *J Neuroocol* 63, 163-171; Rosini P. et al. (2002) Prostate 53, 310-321; Yura Y. (2001) *J. Oral. Pathol. Med.* 30, 159-167]. It has also been shown that FGF activity is linked to the resistance of tumor cells to anticancerous drugs, being possible to revert said resistance by means of FGF inactivation [Song S. et al. (2000) *PNAS* 97, 8658-8663; Pardo OE. et al. (2003) *Mol. Cell.* Biol., 23, 7600-7610; Zhang Y. et al. (2001) *J. Pharmacol. Exp. Ther.* 299, 426-433, Song S. et al. (2001), *Cancer Res.* 61, 6145-6150].

There are numerous antiangiogenic agents for oncology in different stages of clinical development [Krueger et al. (2001) *Seminars in Oncology* 28, 570-576], a considerable number of which are polypeptides that the organism uses to counteract the effect of the positive angiogenesis regulators [Hagedorn, M. & Bikfalvi, A. (2000) *Crit. Rev. Onc. Hemat.* 34, 89-110]. However, when said polypeptides are compared with other compounds having a considerably lower molecular weight, their pharmacological drawbacks are clearly shown.

Polysulfonated binaphthyl ureas known as suramins are considered to be potential antiangiogenic and anticancerous agents [Manetti, F. et al. (2000) *Curr. Pharm. Des.* 6, 1897-1924], at least partially due to their capacity to break the interaction of many growth factors with their membrane receptors, as in the case of FGFs and their tyrosine kinase receptors. Given that it has been shown that heparin breaks aFGF/suramin complexes and counteracts the effect of these polysulfonated ureas, it is thought that suramins act by means of blocking the FGF heparin binding sites.

Another group of antiangiogenic and anticancerous compounds is formed by the suradistas, a type of synthetic derivatives of sulfonic binaphthalene distamycin A. These compounds closely interact with FGFs, inhibit the binding of these polypeptides to the cell membrane tyrosine kinase receptors and suppress FGF-induced angiogenesis and neovascularization *in vivo.*

On the other hand, it has been discovered that 1,3,6-naphthalenetrisulfonic acid (1,3,6-NTS) constitutes a minimum model for aFGF mitogenic activity inhibition by means of suramins and suradistas [Lozano, R.M. et al. (1998) *J. Mol. Biol.* 281, 899-915]. Said compound (1,3,6-NTS) has been tested, with positive results, both in vitro and *in vivo* as an aFGF-induced angiogenesis and glioma proliferation inhibitor [Lozano, R.M. et al. (1998) *J. Mol.* Biol. 281, 899-915; Cuevas, P. et al. (1999) *Neurol. Res.* 21, 191-194; Cuevas, P. et al. (1999) *Neurol. Res.* 21, 481-487; Cuevas, P. et al. (1999) *Neurosci.* Lett. 275, 149-151], suggesting new potential routes for developing new antiangiogenic compounds. The studies by Lozano et al. (mentioned above) also clearly showed that certain naphthalene derivatives containing a reduced number of sulfonate groups per aromatic ring, specifically 1,5-naphthalenedisulfonic acid (1,5-NDS) and naphthalenesulfonic acid (1-NMS), acted as better aFGF mitogenic activity inhibitors than NTS. However, these compounds showed a clear toxicity at concentrations at which they inhibited aFGF mitogenic activity.

Therefore, it is still necessary to find FGF activity inhibiting compounds and, preferably, compounds having said inhibitory capacity and low cellular toxicity.

The inventors of the present invention have observed that certain sulfonic acid derivatives of formula (I), particularly, certain naphthalenesulfonic acid or quinolinesulfonic acid derivatives comprising a sulfonic/sulfonate group and optionally a polar group capable of forming hydrogen bonds, such polar group being an amine group for example, located at certain positions in the aromatic ring, are aFGF-induced mitogenic activity inhibitors on fibroblasts in culture as well as angiogenesis inhibitors, and they further inhibit tumor formation in tests with animals without showing signs of toxicity in the tested animals. Therefore, said compounds are potentially useful in treating cancer, especially in treating solid tumors. Likewise, said sulfonic acid derivatives are also potentially useful in treating other non-tumor angiogenesis-dependent diseases, for example, rheumatoid arthritis, endometriosis, obesity, arteriosclerosis, restenosis, psoriasis, etc.

Therefore, one aspect of this invention is related to a pharmaceutical composition comprising at least one of said naphthalenesulfonic acid or quinolinesulfonic acid derivates, together with a pharmaceutically acceptable excipient.

In another aspect, the present invention is related to the use of said naphthalenesulfonic acid or quinolinesulfonic acid derivatives in preparing a medicinal product for treating cancer, for treating non-tumor angiogenesis-dependent diseases, for example rheumatoid arthritis, endometriosis, obesity, arteriosclerosis, restenosis, psoriasis, etc.

In other additional aspects, this invention is related to the use of the derivatives of formula (I) in preparing a medicinal product to increase cancer cell sensitivity to chemotherapy or radiotherapy, as well as to their use in preparing a diagnostic kit for diseases or conditions linked to FGF biological activity.

Some 2-naphthalenesulfonic acid derivatives are known, for example 5-amino-2-naphthalenesulfonic acid (Aldrich) is used in the dye industry. The sodium salt of said 5-amino-2-naphthalenesulfonic acid is also known [J. Chem. Soc. 3172 (1959); Helv. Chim. Acta 45, 1608 and 1611 (1962)], as well as sodium 5-acetylamino-2-naphthalenesulfonate [J. Med. Chem. 38 (8), 1344-1354 (1995); J. Med. Chem. 40(6), 920-929 (1997)].

### BRIEF DESCRIPTION OF THE FIGURES

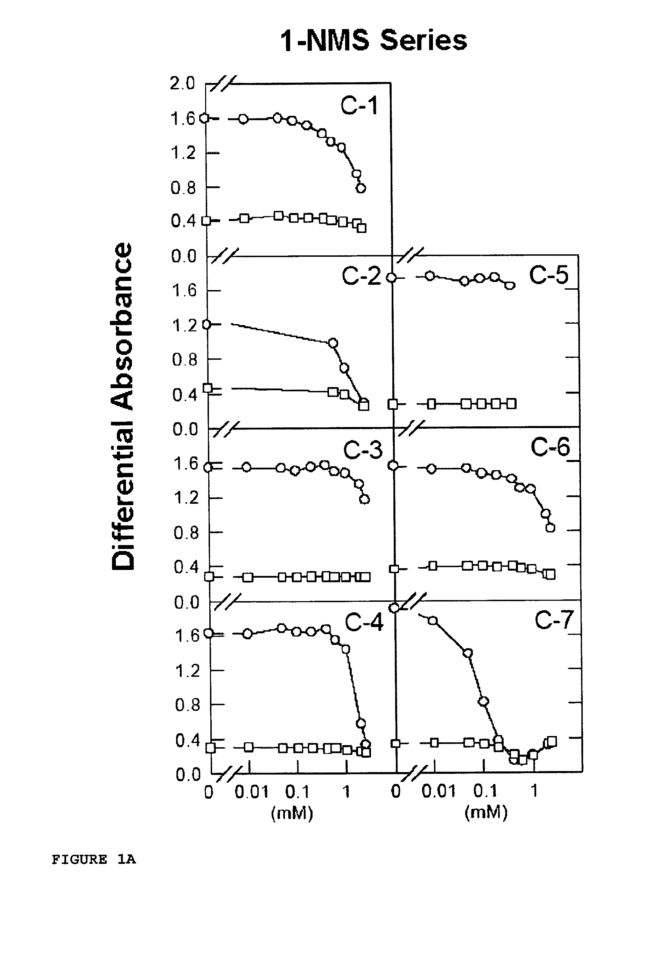

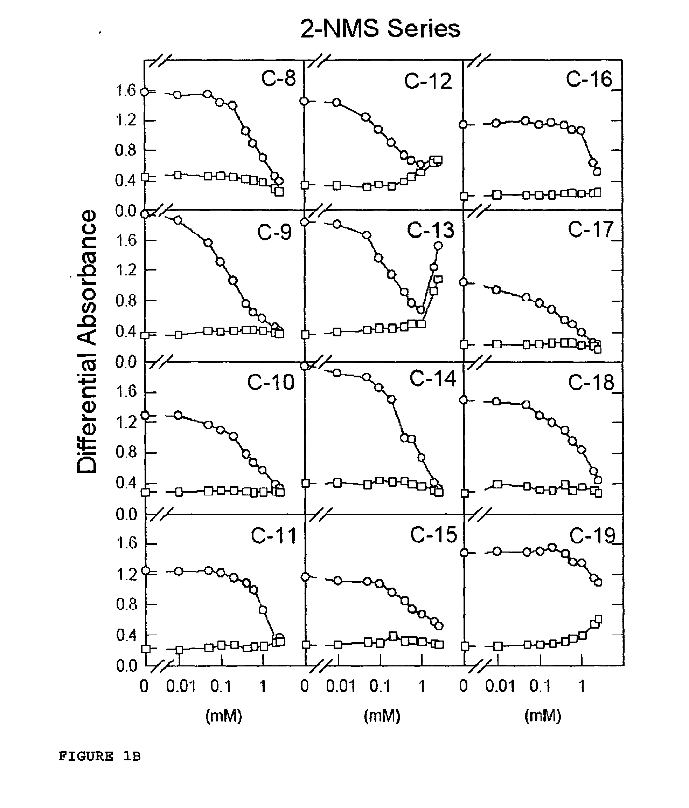

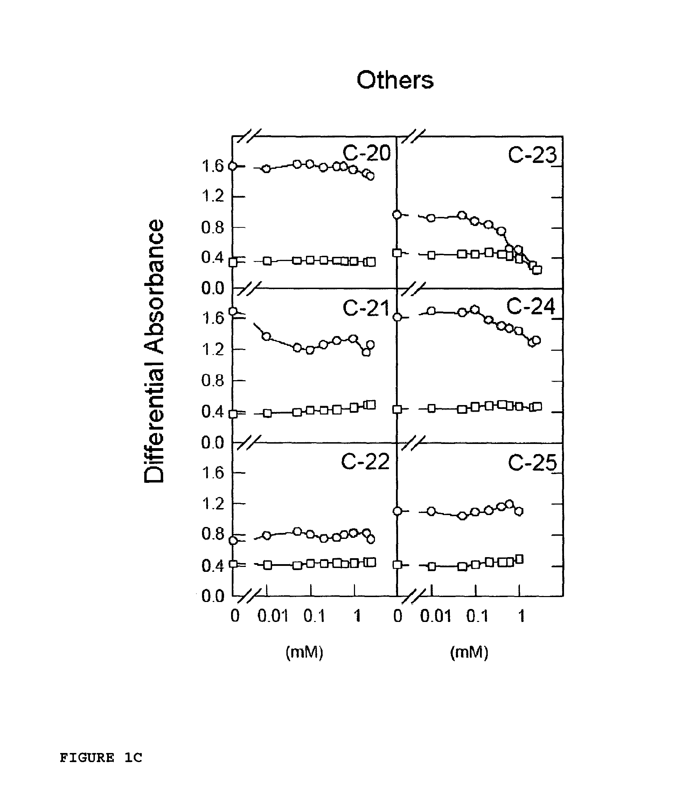

Figures 1A, 1B and 1C are a set of graphs representing the differential absorbance (y-axis) for the concentration (x-axis) of the assayed compound, and they illustrate the effect of increasing concentrations of the C-1 to C-25 compounds: (A) C-1 to C-7; (B) C-8 to C-19; (C) C-20 to C-25; (see Example 1 and Table 1) on aFGF-induced mitogenesis on fibroblasts in culture in minimum medium supplemented with myo-inositol hexasulfate (MIHS) both in the absence of aFGF (□) and in the presence of aFGF (o) .

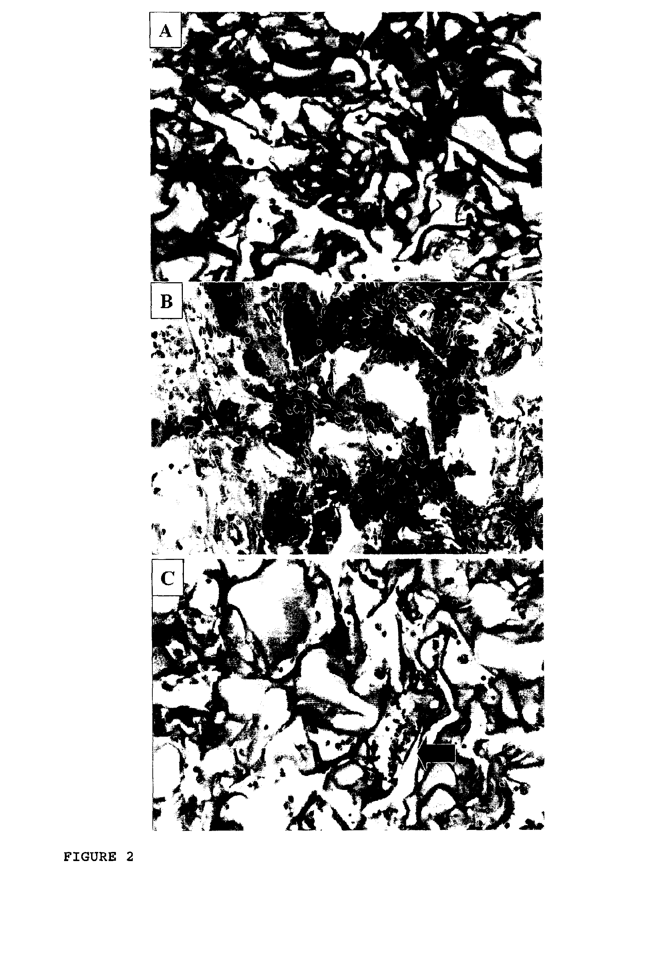

Figure 2 shows illustrative photographs of the antiangiogenic effect of the C-9 compound (see Example 2), which show histological sections representative of sponge implants soaked in PBS (A) and in PBS + aFGF (B and C). The histological sections represented in photograph (C) correspond to sponges implanted in mice intraperitoneally treated with 8 mg/kg of the C-9 compound. The arrow in (C) indicates a vessel containing erythrocytes and leukocytes. Note that the exuberant cellular infiltration shown in (B) disappears in the sponge implants soaked in aFGF and intraperitoneally treated with the C-9 compound. The photographs are shown with an original amplification of a magnification of 50 (50x).

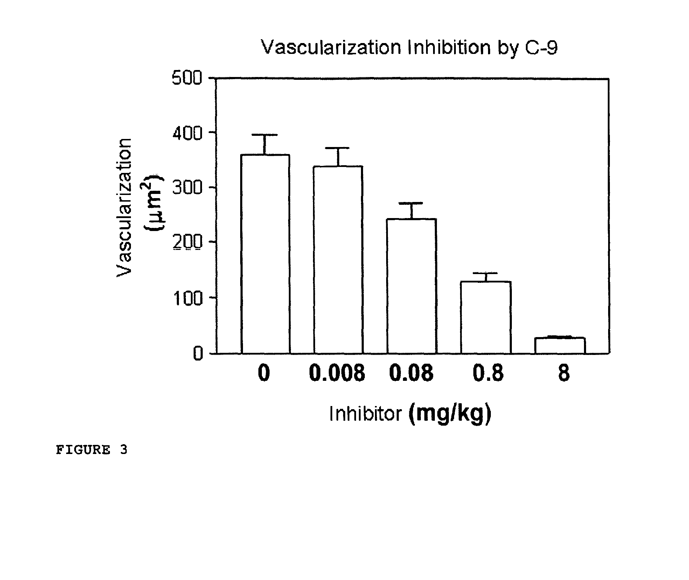

Figure 3 shows a bar graph illustrating vascularization inhibition by the C-9 compound at different concentrations (see Example 2). The inhibitor (C-9) concentration is shown on the x-axis while the measurement of the surface area containing erythrocytes (vascularization) is represented on the y-axis.

### DETAILED DESCRIPTION OF THE INVENTION

In one aspect, the invention is related to a pharmaceutical composition comprising:
(i) at least one compound of formula (I) 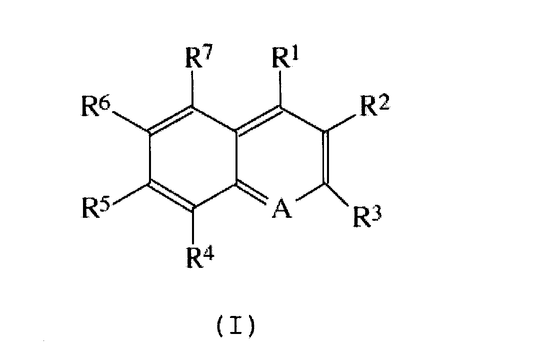 wherein:
   A is N or a CR⁸ formula group;
   R¹, R², R³, R⁴, R⁵, R⁶, R⁷ and R⁸ represent H, SO₃R⁹, OR¹⁰, CO₂R¹⁰, NR¹⁰R¹¹, NH-CO-R¹² or C₁-C₆ alkyl, independently from one another;
   R⁹ is H, ammonium or a cation of an alkali or alkaline-earth metal;
   R¹⁰ and R¹¹ represent H, C₁-C₆ alkyl or C₆-C₁₀ aryl, independently from one another;
   R¹² is OH, C₁-C₆ alkyl or C₆-C₁₀ aryl;
   on the condition that:
   (a) at least one of R¹, to R⁸ is SO₃R⁹, and
   (b) at least one of the remaining R¹ to R³ is an OR¹⁰, CO₂R¹⁰, NR¹⁰R¹¹, NH-CO-R¹² group,
      where R⁹, R¹⁰, R¹¹ and R¹² have the previously mentioned meanings;
      or one of their pharmaceutically acceptable salts or prodrugs; and
(ii) at least one pharmaceutically acceptable excipient.

The term "C₁-C₆ alkyl" as it is used in this description refers to a radical derived from a saturated, linear or branched hydrocarbon of 1 to 6 carbon atoms, for example methyl, ethyl, isopropyl, etc.

The term "C₆-C₁₀ aryl" as it is used in this description refers to a radical derived from an aromatic hydrocarbon of 6 to 10 carbon atoms, for example phenyl, naphthyl, etc.

The term "pharmaceutically acceptable salts or prodrugs" includes any salt, ester, amide, solvate, hydrate, polymorphic form, etc. susceptible to be used in pharmaceutical forms and which is capable of directly or indirectly providing a compound of formula (I) in vivo. The nature of the salt is not critical as long as it is pharmaceutically acceptable.

The salts of the compound of formula (I) can be obtained from organic or inorganic acids or bases by conventional methods well known by a person skilled in the art, by reacting the suitable acid or base with the compound of formula (I).

The prodrugs can be obtained, for example, from a free hydroxyl group converted into an ester or from an amino group converted into an amide by means of processes well known by a person skilled in the art, by reacting the compounds of formula (I) with a carboxylic acid, an anhydride or an acyl halide in the presence of a base or catalyst.

Included among the compounds of formula (I) are naphthalenesulfonic acid derivatives [compounds of formula (I) in which A is CR⁸] and quinolinesulfonic acid derivatives [compounds of formula (I) in which A is nitrogen].

In a particular embodiment, the compound of formula (I) is a naphthalenesulfonic acid derivative belonging to the 2-NMS series (2-naphthalenesulfonic acid derivatives) wherein:
A is CR⁸;
R¹ is H or C₁-C₆ alkyl;
R² is SO₃R⁹;
R³ is H, OH or C₁-C₆ alkyl;
R⁴, R⁵, R⁶, R⁷ and R⁸ represent H, OR¹⁰, CO₂R¹⁰, NR¹⁰R¹¹, NH-COR¹² or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that at least one of R⁴, R⁵, R⁶, R⁷ or R⁸ is OR¹⁰, CO₂R¹⁰, NR¹⁰R¹¹, NH-CO-R¹², where R¹⁰, R¹¹ and R¹² have the previously mentioned meanings.

Preferred compounds of the 2-NMS series include compounds of formula (I) wherein:
A is CR⁸, where R⁸ is H or OH;
R¹ is H;
R² is SO₃R⁹, where R⁹ is H or sodium;
R³ is H; and
R⁴, R⁵, R⁶ and R⁷ represent H, OH, OCH₃, COOH, NH₂, NHCOCH₃, NHCOC₆H₅, NHCH₃ or N(CH₃)₂, independently from one another;
on the condition that at least one of R⁴, R⁵, R⁶ or R⁷ is OH, OCH₃, COOH, NH₂, NHCOCH₃, NHCOC₆H₅ , NHCH₃ or N(CH₃)₂.

Illustrative examples of compounds of the 2-NMS series include:
sodium 5-amino-2-naphthalenesulfonate,
sodium 5-acetylamino-2-naphthalenesulfonate,
sodium 5-benzoylamino-2-naphthalenesulfonate,
sodium 8-amino-2-naphthalenesulfonate,
sodium 8-acetylamino-2-naphthalenesulfonate, and
sodium 4-hydroxy-6-amino-2-naphthalenesulfonate.

Within this 2-NMS series, the sodium 5-amino-2-naphthalenesulfonate, sodium 5-acetylamino-2-naphthalenesulfonate and sodium 4-hydroxy-6-amino-2-naphthalenesulfonate compounds are especially preferred, and particularly sodium 5-amino-2-naphthalenesulfonate, given that they are potent aFGF-induced mitogenic activity inhibitors, aFGF-induced angiogenic activity inhibitors, and furthermore they are not toxic for fibroblasts in culture.

In another particular embodiment, the compound of formula (I) is a naphthalenesulfonic acid derivative belonging to the 1-NMS series (1-naphthalenesulfonic acid derivatives) wherein:
A is CR⁸ ;
R¹ is SO₃R⁹;
R² is H or C₁-C₆ alkyl;
R³ is H, OH or C₁-C₆ alkyl;
R⁴, R⁵, R⁶, R⁷ and R⁸ represent H, OR¹⁰, CO₂R¹⁰, NR¹⁰R¹¹, NH-COR¹² or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that at least one of R⁴, R⁵, R⁶, R⁷ or R⁸ is OR¹⁰, CO₂R¹⁰, NR¹⁰R¹¹, NH-CO-R¹², where R¹⁰, R¹¹ and R¹² have the previously mentioned meanings.

Preferred compounds of the 1-NMS series include compounds of formula (I) wherein:
A is CR⁸;
R¹ is SO₃R⁹, where R⁹ is H or sodium;
R² is H;
R³ is H; and
R⁴, R⁵, R⁶, R⁷ and R⁸ represent H, OH, OCH₃, COOH, NH₂, NHCOCH₃, NHCOC₆H₅, NHCH₃ or N(CH₃)₂, independently from one another;
on the condition that at least one of R⁴, R⁵, R⁶, R⁷ or R⁸ is OH, OCH₃, COOH, NH₂, NHCOCH₃, NHCOC₆H₅, NHCH₃ or N(CH₃)₂.

Illustrative examples of compounds of the 1-NMS series include:
sodium 4-amino-1-naphthalenesulfonate,
sodium 4-acetylamino-1-naphthalenesulfonate,
sodium 4-benzoylamino-1-naphthalenesulfonate,
sodium 5-dimethylamino-1-naphthalenesulfonate, and
sodium 4-amino-3-hydroxy-1-naphthalenesulfonate.

In another particular embodiment, the compound of formula (I) is a quinolinesulfonic acid derivative wherein:
A is N;
R¹, R², R³, R⁴, R⁵, R⁶, R⁷ and R⁸ represent H, SO₃R⁹, OR¹⁰, CO₂R¹⁰, NR¹⁰R¹¹, NH-CO-R¹² or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that:
(a) at least one of R¹ to R⁸ is SO₃R⁹, and
(b) at least one of the remaining R¹ to R⁸ is an OR¹⁰, CO₂R¹⁰, NR¹⁰R¹¹, NH-CO-R¹² group,
   where R⁹, R¹⁰, R¹¹ and R¹² have the previously mentioned meanings.

The composition of the invention may contain one or more compounds of formula (I). The pharmaceutical composition of the invention preferably comprises a compound of formula (I) chosen from sodium 5-amino-2-naphthalenesulfonate, sodium 5-acetylamino-2-naphthalenesulfonate, sodium 5-benzoylamino-2-naphthalenesulfonate, and mixtures thereof.

The compounds of formula (I) are known products which can be obtained either commercially or by means of conventional methods (Example 4).

The compounds of formula (I) have the capacity to inhibit FGF activity both in vitro and *in vivo,* FGF being one of the key substances for inducing vascular network formation, as well as to inhibit angiogenesis induced by implanted tumors. Said compounds of formula (I) have the capacity to inhibit tumor formation in tests with animals. In particular, certain compounds of formula (I) have antiangiogenic activity together with a low cytotoxicity, which makes them very appropriate as candidates for treating cancer, especially in treating solid tumors, and as candidates for treating non-tumor angiogenesis-dependent diseases. Though not intended to be linked to any theory, it is believed that the compounds of formula (I) prevent the formation of the vascular network that is induced when solid tumors become malignant, thereby causing the tumor to collapse due to a lack of nutrients and autointoxication, in addition to directly inhibiting tumor cell proliferation.

Several tests clearly show the capacity of the compounds of formula (I) to inhibit aFGF-induced mitogenic activity on fibroblasts in culture (Example 1), as well as to inhibit in vivo aFGF and bFGF-induced angiogenesis (Example 2), and to inhibit angiogenesis induced in animals by tumor cell implantation (Example 3).

The pharmaceutical composition provided by this invention comprises a therapeutically effective amount of at least one compound of formula (I) together with at least one pharmaceutically acceptable excipient. Said pharmaceutical composition is useful for administration and/or application in or on the body of a mammal, preferably a human being.

The use of the compounds of formula (I) in preparing said pharmaceutical composition is a further aspect of this invention.

The compounds of formula (I) can be administered to treat cancer, especially solid tumors or, alternatively, non-tumor angiogenesis-dependent diseases, for example rheumatoid arthritis, endometriosis, obesity, arteriosclerosis, restenosis or psoriasis, by any means allowing contact between the compound of formula (I) and the action site thereof on the body of a mammal. The therapeutically effective amount of the compound of formula (I) that must be administered as well as its dosage for treating a pathological condition with said compounds of formula (I) and/or pharmaceutical compositions of the invention will depend on several factors, including among these factors the disease to be treated, age, patient condition, seriousness of the disease, the administration method and frequency, the compound of formula (I) to be used, etc.

Pharmaceutical compositions containing the compounds of formula (I) provided by this invention may be presented in any administration form that is considered appropriate, for example, a solid or liquid, and they may by administered by any suitable method, for example orally, parenterally, rectally or topically, to which end they will include the pharmaceutically acceptable excipients necessary for the formulation of the desired administration form. A review of different dosage forms for administering medicinal products and the excipients necessary for obtaining them can be found, for example, in the "Tratado de Farmacia Galénica", C. Faulí i Trillo, 1993, Luzán 5, S.A. Ediciones, Madrid.

Said compounds of formula (I) can be used in preparing a medicinal product for treating cancer, especially solid tumors [Rosti G. et al. (2002) *Crit. Rev. Oncol. Hematol.* 41, 129-240], for example breast cancer, prostate cancer, etc. Alternatively, the compounds of formula (I) can be used in preparing a medicinal product for treating other angiogenesis-dependent diseases, for example skin diseases, rheumatoid arthritis, endometriosis, obesity, arteriosclerosis, restenosis, psoriasis, etc.

Other active agents, for example anticarcinogenic agents, can be used together with a compound of formula (I). The different active agents can be administered simultaneously in a single formulation or in several, or sequentially.

The use of the derivatives of formula (I) in preparing a medicinal product for increasing cancer cell sensitivity to chemotherapy or radiotherapy as well as their use in preparing a diagnostic kit for diseases or conditions linked to FGF biological activity are other further aspects of the present invention.

The following examples illustrate the invention and must not be considered as limiting on the scope thereof.

### EXAMPLE 1

### aFGF-Induced Mitogenic Activity Inhibition

The works of Lozano et al. [Lozano, R.M. et al. (1998) *J. Mol. Biol.* 281, 899-915] clearly showed that certain naphthalenesulfonic compounds, such as 1,3,6-naphthalenetrisulfonic acid (1,3,6-NTS), 1,5-naphthalenedisulfonic acid (1,5-NDS) and 1-naphthalenesulfonic acid (1-NMS) are aFGF-induced mitogenic activity inhibitors. 1-NMS was a more potent inhibitor than 1,3,6-NTS and 1,5-NDS. However, 1-NMS and 1,5-NDS are toxic at concentrations at which they inhibit aFGF-induced mitogenic activity.

Now, for the purpose of finding other arylsulfonic compounds with better potential pharmacological profiles, an iterative test-error process has been followed by means of which certain functional groups with a different steric volume or load were placed in different positions of different aromatic compounds, testing their aFGF-induced mitogenesis inhibitory activity on fibroblasts in culture in the presence of an aFGF activator. The materials used as starting materials in the search for said arylsulfonic compounds that inhibit aFGF-induced mitogenic activity were 1-naphthalenesulfonic acid (1-NMS), which gave rise to the series called 1-NMS, and 2-naphthalenesulfonic acid (2-NMS), which gave rise to the series called 2-NMS. Like 1-NMS, 2-NMS inhibits aFGF-induced mitogenic activity (although it does so at lower concentrations) and is toxic for quiescent fibroblasts within the concentration range in which it inhibits aFGF-induced mitogenic activity. Other arylsulfonic compounds were also tested (see Table 1).

### 1.1 Tested Compounds

Table 1 lists all the tested compounds, while their preparation is described in Example 4.

**Table 1**

| Tested Compounds | |
|---|---|
| Code | Chemical Name |
| | 1-NMS Series |
| C-1 | 1-naphthalenesulfonic acid |
| C-2 | sodium 2-methyl-1-naphthalenesulfonate |
| C-3 | sodium 4-amino-1-naphthalenesulfonate |
| C-4 | sodium 4-acetylamino-1-naphthalenesulfonate |
| C-5 | sodium 4-benzoylamino-1-naphthalenesulfonate |
| C-6 | sodium 5-dimethylamino-1-naphthalenesulfonate |
| C-7 | sodium 4-amino-3-hydroxy-1-naphthalenesulfonate |

| | 2-NMS Series |
|---|---|
| C-8 | 2-naphthalenesulfonic acid |
| C-9 | sodium 5-amino-2-naphthalenesulfonate |
| C-10 | sodium 5-acetylamino-2-naphthalenesulfonate |
| C-11 | sodium 5-benzoylamino-2-naphthalenesulfonate |
| C-12 | sodium 2-{[(6-sulfo-1-naphthyl)amino]carbonyl}benzoic acid |
| C-13 | sodium 5-({[(6-oxo-6[(6-sulfo-1-naphthyl)amino]hexanoyl}amino)2-naphthalenesulfonate |
| C-14 | sodium 8-amino-2-naphthalenesulfonate |
| C-15 | sodium 8-acetylamino-2-naphthalenesulfonate |
| C-16 | sodium 8-benzoylamino-2-naphthalenesulfonate |
| C-17 | sodium 6-amino-4-hydroxy-2-naphthalenesulfonate |
| C-18 | sodium 7-amino-4-hydroxy-2-naphthalenesulfonate |
| C-19 | sodium 7-acetylamino-4-hydroxy-2-naphthalenesulfonate |

| | Others |
|---|---|
| C-20 | sodium 4-aminobenzenesulfonate |
| C-21 | sodium 4-(aminomethyl)benzenesulfonate |
| C-22 | sodium 4-(aminoethyl)benzenesulfonate |
| C-23 | sodium 6-quinolinesulfonate |
| C-24 | sodium 8-quinolinesulfonate |
| C-25 | 2-[(dimethylamino)methyl]-1H-indole-5-sulfonic acid |

### 1.2 aFGF-Induced Mitogenesis Inhibition Test

The mitogenic response of aFGF on cell cultures of Balb/c 3T3 fibroblasts [ATCC CRL-1658], the cell line normally used to evaluate mitogenic activity of said aFGF protein, was evaluated by means of a conventional process [Lozano, R.M. et al. (1998) *J*. *Mol.* Biol. 281, 899-915], briefly consisting in placing increasing concentrations of the compound to be tested in contact with fibroblasts in culture, for 60 hours, in minimum medium supplemented with myo-inositol hexasulfate (MIHS) (20 µg/mL) in the absence and in the presence of aFGF (0.32 ng/mL) [the aFGF of 139 amino acid residues disclosed by Zazo M., Lozano R.M., Ortega S., Varela J., Diaz-Orejas R., Ramirez J.M., Giménez-Gallego G., in *Gene* (1992) 113, 231-238, was used].

### 1.3 Results

The obtained results are shown in Figure 1, which summarizes the results of the aFGF-induced mitogenesis inhibition tests on fibroblasts in culture for all the tested compounds (o), together with the toxicity analyses for said compounds for quiescent fibroblasts (□).

Briefly, said results clearly show that virtually all the tested compounds progressively inhibit aFGF-induced mitogenesis at concentrations exceeding 1 mM (see the decrease in the differential absorbance units (o) as the concentration of the tested compound increases). The absence of floating cells or other cell rupture signals in said mitogenic tests also indicates that many of the tested compounds are not toxic for fibroblasts in the absence or in the presence of aFGF. On the other hand, since aFGF-induced mitogenesis inhibition by means of the use of the tested compounds may be compensated by raising the heparin concentrations (an aFGF mitogenic activity activator) (data not shown), it can be asserted that said compounds are aFGF-induced mitogenic activity inhibitors which act by blocking the binding between the protein and its activator.

More specifically, with regard to the 1-NMS series, it can be observed that the C-1, C-2, C-4, C-6 and C-7 compounds are aFGF-induced mitogenic activity inhibitors even though they show a certain toxic effect on quiescent fibroblasts within the concentration range in which they inhibit aFGF mitogenic activity. Compound C-7 is the one that shows the lowest IC₅₀, though the opposite effect is observed at concentrations exceeding 0.2 mM (that is, the proliferation of quiescent fibroblasts cultured in the absence of aFGF significantly increases). Compounds C-3 and C-5 show a low aFGF-induced mitogenesis inhibitory activity within the tested concentration range although they are not toxic for the quiescent fibroblasts within said concentration range.

With respect to the 2-NMS series, it is observed that compound C-8 inhibits aFGF-induced mitogenic activity at lower concentrations than compound C-1; however, it is also toxic for quiescent fibroblasts within the concentration range in which it inhibits aFGF-induced mitogenic activity. Compounds C-12 and C-13 are also effective aFGF-induced mitogenesis activity inhibitors, although they have a high mitogenesis inductor activity even in the absence of aFGF. However, this last effect does not occur in compound C-9 which had the second best IC₅₀ value, nor was the toxic effect described for compounds C-1 and C-8 on the quiescent fibroblasts observed, at least in the concentration range in which it inhibits aFGF-induced mitogenic activity. Two other compounds with an inhibitory activity that is slightly less than that of C-9 have further been found, specifically compounds C-10 and C-17 which, like C-9, were innocuous for the quiescent fibroblasts. Compounds C-15 and C-16 are aFGF-induced mitogenic activity inhibitors and are innocuous for quiescent fibroblasts within the tested concentration range. Compounds C-11, C-14 and C-18 are also aFGF-induced mitogenic activity inhibitors, although compound C-14 shows a certain toxic effect on quiescent fibroblasts within the tested concentration range. Compound C-19 shows a certain tumorigenic effect on quiescent fibroblasts. The inhibitory activity IC₅₀ of compound C-9 is 265 µm, exceeding that of 1,3,6-NTS by more than two orders of magnitude [Lozano, R.M. et al. (1998) *J. Mol. Biol.* 281, 899-915] and was chosen to evaluate its use as an angiogenesis inhibitor in vivo by means of a conventional angiogenesis test in mice (Example 2) and as an inhibitor of tumor formation in animals (Example 3).

Compounds C-20, C-22 and C-24 showed little aFGF-induced mitogenesis inhibitory activity at the tested concentrations, although they were innocuous for quiescent fibroblasts. Compound C-23 is a potent aFGF-induced mitogenic activity inhibitor although it shows a toxic effect on quiescent fibroblasts within the concentration range in which it inhibits aFGF-induced mitogenic activity. Compounds C-21 and C-25 show a certain tumorigenic effect on quiescent fibroblasts.

The obtained results allow establishing a series of stereochemical guidelines that improve the potential pharmacological applicability of arylsulfonic derivatives, particularly naphthalenesulfonic acid derivatives, in angiogenesis, specifically:
a) the sulfonate group must preferably be located in position 2 of naphthalene since the 2-NMS series derivatives are generally more potent aFGF-induced mitogenic activity inhibitors than the 1-NMS series derivatives;
b) among the 2-NMS series derivatives, those containing an amino group in position 5 or 6 of the naphthalene ring are better aFGF-induced mitogenic activity inhibitors (see C-9 and C-17 vs. C-14 and C-18, respectively); and
c) the size of the functional group present in position 5 seems to have certain relevance since the substitution of an amino group by a small amide does not substantially alter the inhibitory activity (see C-9 vs. C-10); however, a significant reduction is observed when a bulky amide is introduced (see C-9 vs. C-11 and C-13).

Although the naphthalene ring seems to constitute the nucleus of the most suitable inhibitor, it is to be expected that quinolines with sulfonic groups in position 2 and amine groups in position 5 or 6 can also be used.

Compound C-7 constitutes an exception to the previously mentioned rules since it is the compound that showed the best aFGF-induced mitogenic activity inhibitory activity, although it is toxic for quiescent fibroblasts.

### EXAMPLE 2

### Angiogenesis Inhibition Test in Mice

A standard angiogenesis test was performed with mice in order to evaluate the C-9 compound as an angiogenesis inhibitor.

Pathogen-free C57/BI/6 mice (Charles River, Spain) weighing 25 + 4 g were used. The animals were kept in plastic cages under controlled temperature and humidity conditions; they had water and food *ad libitum* and a schedule of 12 hours of daylight and darkness was maintained. The guidelines on animal welfare of the NIH and the European Union were followed meticulously.

10 mm long sterile gelatin sponge cubes (Curaspon Dental, Clinimed Holding, Zwanenburg, Holland) were subcutaneously implanted in the backs of the mice after inducing intraperitoneal anesthesia [Cuevas, P. et al. (1999) *Neurol. Res.* 21, 191-194]. The animals were distributed into 2 groups: Group A (n=10): formed by animals in which sponges loaded with 200 µL of phosphate buffered saline (PBS) containing 29 µg/mL of heparin were implanted; and Group B (n= 40) : formed by animals in which sponges soaked with the same solution as that of Group A but containing 10 µg/mL of aFGF were implanted. The skin was sutured after implanting the sponge in the subcutaneous bursa. The mice in Group B were randomly divided into four groups (n= 10) that received 0.008, 0.08, 0.8 and 8 mg/kg of compound C-9, respectively, by means of intraperitoneal injection of 200 µL of PBS 24 hours after surgical treatment [Pesenti, E. et al. (1992) *Brit*. *J. Cancer* 66, 367-372; Cuevas, P. et al. (1999) *Neurol. Res*. 21, 191-194]. All the procedures were performed under sterile conditions.

In order to evaluate angiogenesis, the mice were again anesthetized as previously described, and the sponges were surgically extracted, which were treated to perform histological studies by means of conventional methods [Cuevas, P. et al. (1999) *Neurol. Res.* 21, 191-194] 7 days after the implants. Neovascularization was quantified by means of a morphometric computer program connected to a microscope. The growth of new vessels inside the sponges was evaluated by means of the measurement of the surface area containing erythrocytes. Neovascularization was analyzed in 4 predetermined visual fields of 3 different sections at a magnification of 10. The statistical analyses were performed using Student's t-Test.

Figures 2 and 3 illustrate the neovascularization inhibition induced by means of aFGF in gelatin sponges implanted subcutaneously in mice treated with C-9. The calculation of the number of new blood vessels per unit of area with different doses of the C-9 compound clearly showed that neovascularization inhibition was already assessable in mice that received 0.008 and 0.08 mg/kg and was virtually complete with doses exceeding 0.8 mg/kg. This data clearly shows that C-9 is a considerably more effective neovascularization inhibitor than suramines and 1,3,6-NTS since in these last 2 cases, it seems that concentrations of about 200 mg/kg are required to reach substantial neovascularization inhibition [Pesenti, E. et al. (1992) Brit. *J. Cancer* 66, 367-372; Cuevas, P. et al. (1999) *Neurol. Res.* 21, 191-194]. Equivalent results were obtained when aFGF was substituted with bFGF (data not shown). The highest tested C-9 concentrations did not cause any toxic death or apparent alterations in the animals, nor did it cause weight changes.

### EXAMPLE 3

### Angiogenesis and Tumor Growth Inhibition in Animals with Implanted Tumors

### 3.1 Test with Albino Rabbits

Albino New Zealand rabbits (3 ± 0.5 kg, male and female in equal numbers) were used and were implanted with 5 µL of a glioma C6 cell suspension [ATCC CCL-107] subepithelially injected into the cornea with a 10 µL Hamilton syringe using a surgical microscope. The injection is located at 2 mm from the limbal margin of the cornea. At the same time, an osmotic minipump was subcutaneously implanted in the neck region for the purpose of assuring continuous infusion of the compound to be tested (C-9) or of a saline solution (control). A catheter guided the solution to the sclera at a constant rate of 0.2 mg/h. It is thus calculated that a concentration of about 0.003 mg/mL of the compound to be tested or saline solution is maintained in the cornea. Treatment was maintained for 14 days. The corneas were eliminated at the end of each experiment and at fixed intervals after each treatment and processed for histological examination.

A very positive apoptosis index was observed (apoptosis increases with the number of treatment days) and a very reduced corneal neovascularization area (in comparison with the untreated animals). The following could be confirmed by means of microscopic observation: a lower number of glioma C6 cells, a higher number of apoptotic bodies, the presence of broken blood vessels and the presence of apoptotic endothelial nuclei in treated animals.

### 3.2 Tests with Rats

A test similar to the one described in Example 3.1 was performed with rats, implanting the C6 glioma cells in the caudal area of the brain and the osmotic micropump in the back of the rats. A catheter led the treatment solution that contained the C-9 compound or the saline solution to the area of the glioma implant. Coronal sections of the brain of the rats thus treated were obtained and compared with coronal sections of the brain of untreated rats. The analysis was done by means of NMR spectroscopy imaging.

In this case, it could be seen that the tumor mass significantly decreased. Intratumor vascularization inhibition was also confirmed (by a factor of six). The "apoptotic index" was also obtained, which clearly showed that the C-9 compound was a potent apoptosis inhibitor in experimental gliomas.

### EXAMPLE 4

### Preparation of the Tested Compounds

Unless otherwise indicated, the tested compounds were obtained from commercially available sources and used with no further purification. The structure and purity of the prepared compounds was confirmed by means of NMR. The solvents were dried and purified using conventional methods. Particularly compounds C-1 and C-3 and the acid precursors of compounds C-6, C-7, C-9, C-14, C-17, C-18, C-20, C-21 and C-22 are commercial products. The remaining tested compounds were obtained by means of conventional methods, as briefly described below.

Generally, all the reactions were performed in dry flasks fitted with a glass stopper or with a rubber septum under positive argon pressure, unless otherwise indicated. The liquids sensitive to air and to humidity and the solutions were transferred by syringe or stainless steel cannula. The flash chromatography column was prepared using silica gel of 230-400 mesh diameter. Thin layer chromatography was carried out in Kieselgel 60 F₂₅₄ (Merck). The detection was first carried out by means of UV (245 mm) and then, after treatment with a 20% aqueous sulfuric acid solution (200 mL), in acetic acid (800 mL). Anhydrous MgSO₄ or Na₂SO₄ were used to dry the organic solutions. The elimination of the solvents was carried out under vacuum conditions with a rotary evaporator.

### Sodium Salts

The sodium salts were prepared by adding the required amount of 0.1 N sodium hydroxide to a suspension of the corresponding sulfonic acid derivative in water. Evaporation of the solvents provided the sodium salts.

### Acetylamide Derivatives (C-4, C-10, C-15, C-19)

Acetylamide derivatives were prepared by heating a suspension of the corresponding sodium salts (C-3, C-9, C-14 and C-18) in acetic anhydride for 4 hours at 90°C. The solvent was evaporated, producing the acetylamide derivatives.

### Benzoylamide Derivatives (C-5, C-11, C-16)

A slight excess of benzoyl chloride (1.2 equivalents) was added to a solution of the corresponding sulfonic acid in pyridine. The solvent was evaporated after 30 minutes and the residue was chromatographed on silica gel column (1:9 MeOH:CH₂Cl₂ v/v) . The purified product was transformed into its corresponding sodium salts.

### Compound C-2

Concentrated H₂SO₄ (0.32 ml) was added to 2-methylnaphthalene (2.8 mmoles). The mixture was heated in an oil bath at 80°C for 3 hours. The solution was cooled, poured over ice and basified by means of adding 1N NaOH. The product was separated under cold conditions and isolated by filtration.

### Compound C-8

Ion exchange resin (Amberlite) (H⁺ form) was added to a solution of sodium 2-naphthalenesulfonate in methanol. The mixture was stirred overnight and filtered and the solvent was evaporated. The residue was lyophilized yielding 2-naphthalenesulfonic acid.

### Compound C-12

An excess of phthalic anhydride (1.3 equivalents) was added to a solution of C-9 (0.6 mmoles) in methanol. The mixture was stirred overnight and evaporated. The residue was chromatographed on silica gel column (3:7 MeOH:CH₂Cl₂ v/v) to produce the required product.

### Compound C-13

Adipoyl chloride (0.5 equivalents) was added to a suspension of C-9 (0.1 mmoles) in pyridine. The stirring was continued for 48 hours and the solvent was evaporated. The residue was digested with a small volume of methanol and the solid that is separated was collected and characterized.

### Compound C-23

Concentrated sulfuric acid (61 mL) is added in portions over a mixture of sulfanilic acid (75 g, 390 mmoles), glycerol (103 mL, 1.41 moles) and nitrobenzene (20 mL, 195 mmoles), then the reaction mixture is carefully heated to about 80°C. The exothermal process is then triggered, spontaneously evolving up to 120-140°C. Then the external heating is stopped and once the reaction is stabilized (after about 30 minutes), it is heated again externally up to 140-145°C for 4 hours. After this time has lapsed, the reaction mixture is cooled and poured over 500 ml of a water-ice mixture, keeping it in the refrigerator at +4°C for 4 days. Once this time has lapsed, the little solid generated is eliminated and the filtration waters are diluted 6 times their own volume in water and treated with BaCO₃, again filtering the solid residue generated. The filtration waters are treated with 20% NaOH up to a pH of 11. The solvent of an aliquot of these alkali waters is eliminated under vacuum conditions and is purified by means of chromatography column on silica gel (Merck, Kieselgel H F₂₅₄, 7:3 AcOEt/MeOH) , isolating an analytically pure sample of the sodium salt.

### Compound C-24

Quinoline (9 mL, 76.2 mmoles) was added dropwise over a flask containing H₂SO₄ SO₃ (20% SO₃, 30 mL). The temperature is maintained under 50°C during the entire process. Once the addition has ended, the mixture is heated at 140°C for 2 hours. The cooled raw product is then poured over a water-ice mixture, isolating 8-sulfoquinoline as a white solid after abundant washing with water. Once dried under vacuum conditions, 8.25 g of acid (> 50%) are quantified. This acid was used to prepare the corresponding sodium salt as previously indicated.

### Compound C-25

6.45 mmoles of gramine were dissolved in 10 mL of concentrated H₂SO₄ and stirred for 5 minutes at 0°C. The solution was poured over ice and basified by means of adding 1N NaOH. This aqueous solution was extracted with dichloromethane, neutralized by means of adding 1N H₂SO₄ and evaporated. The residue was digested with hot ethanol and the solids separated by filtration. The ethanol solution was evaporated and the residue was subjected to column chromatography (ethanol) in order to produce the desired compound.

## Claims

1. A pharmaceutical composition comprising:
(i) at least one compound of formula (I) 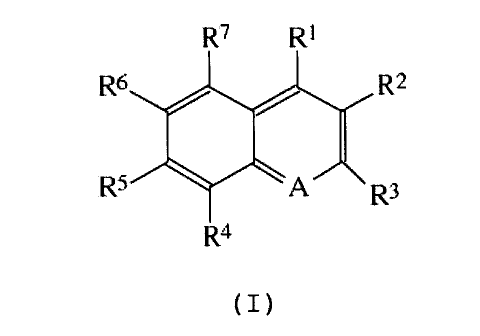 wherein:
A is N or a CR⁸ formula group;
R¹, R², R³, R⁴, R⁵, R⁶, R⁷ and R⁸ represent H, SO₃R⁹, OR¹⁰, CO₂R¹⁰, NR¹⁰R¹¹, NH-CO-R¹², or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that:
(a) at least one of R¹ to R⁸ is SO₃R⁹, and
(b) at least one of the remaining R¹ to R⁸ is an OR¹⁰, CO₂R¹⁰, NR¹⁰R¹¹, NH-CO-R¹² group,
where R⁹, R¹⁰, R¹¹ and R¹² have the previously mentioned meanings;
or one of their pharmaceutically acceptable salts or prodrugs; and
(ii) at least one pharmaceutically acceptable excipient.

2. A pharmaceutical composition according to claim 1, comprising a compound of formula (I) wherein:
A is CR⁸;
R¹ is H or C₁-C₆ alkyl;
R² is SO₃R⁹;
R³ is H, OH or C₁-C₆ alkyl;
R⁴, R⁵, R⁶, R⁷ and R⁸ represent H, OR¹⁰, CO₂R¹⁰, NR¹⁰R¹¹, NH-COR¹² or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that at least one of R⁴, R⁵, R⁶, R⁷ or R⁸ is OR¹⁰, CO₂R¹⁰, NR¹⁰R¹¹, NH-CO-R¹², where R¹⁰, R¹¹ and R¹² have the previously mentioned meanings.

3. A pharmaceutical composition according to claim 2, comprising a compound of formula (I) wherein:
A is CR⁸, where R⁸ is H or OH;
R¹ is H;
R² is SO₃R⁹, where R⁹ is H or sodium;
R³ is H; and
R⁴, R⁵, R⁶ and R⁷ represent H, OH, OCH₃, COOH, NH₂, NHCOCH₃, NHCOC₆H₅, NHCH₃ or N(CH₃)₂, independently from one another;
on the condition that at least one of R⁴, R⁵, R⁶ or R⁷ is OH, OCH₃, COOH, NH₂, NHCOCH₃, NHCOC₆H₅ , NHCH₃ or N(CH₃)₂.

4. A Pharmaceutical composition according to claims 2 or 3, comprising a compound of formula (I) chosen from the group formed by sodium 5-amino-2-naphthalenesulfonate, sodium 5-acetylamino-2-naphthalenesulfonate, sodium 5-benzoylamino-2-naphthalenesulfonate, sodium 8-amino-2-naphthalenesulfonate, 8-acetylamino-2-naphthalenesulfonic acid, sodium 4-hydroxy-6-amino-2-naphthalenesulfonate and mixtures thereof.

5. A pharmaceutical composition according to claim 1, comprising a compound of formula (I) wherein:
A is CR⁸;
R¹ is SO₃R⁹;
R² is H or C₁-C₆ alkyl;
R³ is H, OH or C₁-C₆ alkyl;
R⁴, R⁵, R⁶, R⁷ and R⁸ represent H, OR¹⁰, CO₂R¹⁰, NR¹⁰R¹¹, NH-COR¹² or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that at least one of R⁴, R⁵, R⁶, R⁷ or R⁸ is OR¹⁰, CO₂R¹⁰, NR¹⁰R¹¹, NH-CO-R¹², where R¹⁰, R¹¹ and R¹² have the previously mentioned meanings.

6. A pharmaceutical composition according to claim 5, comprising a compound of formula (I) wherein:
A is CR⁸;
R¹ is SO₃R⁹, where R⁹ is H or sodium;
R² is H;
R³ is H; and
R⁴, R⁵, R⁶, R⁷ and R⁸ represent H, OH, OCH₃, COOH, NH₂, NHCOCH₃, NHCOC₆H₅, NHCH₃ or N(CH₃)₂, independently from one another;
on the condition that at least one of R⁴, R⁵, R⁶, R⁷ or R⁸ is OH, OCH₃, COOH, NH₂, NHCOCH₃, NHCOC₆H₅, NHCH₃ or N(CH₃)₂.

7. A pharmaceutical composition according to claims 5 or 6 comprising a compound of formula (I) chosen from the group formed by sodium 4-amino-1-naphthalenesulfonate, sodium 4-acetylamino-1-naphthalenesulfonate, sodium 4-benzoylamino-1-naphthalenesulfonate, sodium 5-dimethylamino-1-naphthalenesulfonate, sodium 4-amino-3-hydroxy-1-naphthalenesulfonate and mixtures thereof.

8. A pharmaceutical composition according to claim 1, comprising a compound of formula (I) wherein:
A is N;
R¹, R², R³, R⁴, R⁵, R⁶, R⁷ and R⁸ represent H, SO₃R⁹, OR¹⁰, CO₂R¹⁰, NR¹⁰R¹¹, NH-CO-R¹² or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that:
(a) at least one of R¹ to R⁸ is SO₃R⁹, and
(b) at least one of the remaining R¹ to R⁸ is an OR¹⁰, CO₂R¹⁰, NR¹⁰R¹¹, NH-CO-R¹² group,
where R⁹, R¹⁰, R¹¹ and R¹² have the previously mentioned meanings.

9. A pharmaceutical composition according to claim 1, comprising one or more compounds of formula (I).

10. Use of a naphthalenesulfonic acid derivative of formula (I) 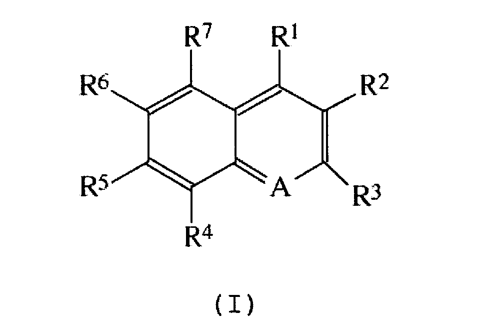 wherein:
A is N or a CR⁸ formula group;
R¹, R², R³, R⁴, R⁵, R⁶, R⁷ and R⁸ represent H, SO₃R⁹, OR¹⁰, CO₂R¹⁰, NR¹⁰R¹¹, NH-CO-R¹², or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that:
(a) at least one of R¹ to R⁸ is SO₃R⁹, and
(b) at least one of the remaining R¹ to R⁸ is an OR¹⁰, CO₂R¹⁰, NR¹⁰R¹¹, NH-CO-R¹² group,
where R⁹, R¹⁰, R¹¹ and R¹² have the previously mentioned meanings;
or one of their pharmaceutically acceptable salts or prodrugs in preparing a medicinal product for preventing or treating a disease or condition in which FGF biological activity is involved.

11. Use according to claim 10, wherein the disease or condition is an angiogenesis-dependent disease chosen from rheumatoid arthritis, endometriosis, obesity, arteriosclerosis, restenosis or psoriasis.

12. Use according to claim 10, wherein the disease or condition is any type of cancer.

13. Use of a compound of formula (I) 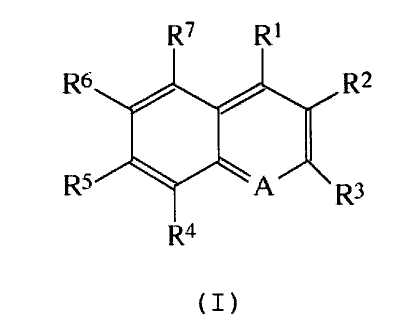 wherein:
A is N or a CR⁸ formula group;
R¹, R², R³, R⁴, R⁵, R⁶, R⁷ and R⁸ represent H, SO₃R⁹, OR¹⁰, CO₂R¹⁰, NR¹⁰R¹¹, NH-CO-R¹², or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that:
(a) at least one of R¹ to R⁸ is SO₃R⁹, and
(b) at least one of the remaining R¹ to R⁸ is an OR¹⁰, CO₂R¹⁰, NR¹⁰R¹¹, NH-CO-R¹² group,
where R⁹, R¹⁰, R¹¹ and R¹² have the previously mentioned meanings;
or one of their pharmaceutically acceptable salts or prodrugs in preparing a medicinal product for increasing cancer cell sensitivity to chemotherapy or radiotherapy.

14. Use of a compound of formula (I) 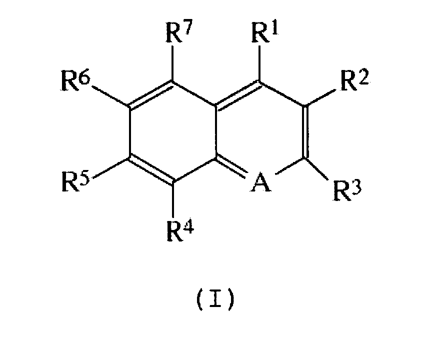 wherein:
A is N or a CR⁸ formula group;
R¹, R², R³, R⁴, R⁵, R⁶, R⁷ and R⁸ represent H, SO₃R⁹, OR¹⁰, CO₂R¹⁰, NR¹⁰R¹¹, NH-CO-R¹², or C₁-C₆ alkyl, independently from one another;
R⁹ is H, ammonium or a cation of an alkali or alkaline-earth metal;
R¹⁰ and R¹¹ represent H, C₁-C₆ alkyl or C₆-C₁₀ aryl, independently from one another;
R¹² is OH, C₁-C₆ alkyl or C₆-C₁₀ aryl;
on the condition that:
(a) at least one of R¹ to R⁸ is SO₃R⁹, and
(b) at least one of the remaining R¹ to R⁸ is an OR¹⁰, CO₂R¹⁰, NR¹⁰R¹¹, NH-CO-R¹² group,
where R⁹, R¹⁰, R¹¹ and R¹² have the previously mentioned meanings;
or one of their pharmaceutically acceptable salts or prodrugs in preparing a diagnostic kit for diseases or conditions linked to FGF biological activity.
